# EUROPEAN PATENT APPLICATION

(11) **EP 3 309 256 A1**
(43) Date of publication of application: **18.04.2018**
(21) Application number: 16806941.7
(22) Date of filing: 10.06.2016
(51) Int. Cl.: C12N 15/82

(54) **USE OF TWO ISOLATES OF PEPINO MOSAIC VIRUS TO PROTECT AGAINST INFECTION BY THE SAME VIRUS**

(30) Priority: 12.06.2015 ES 201530824
(71) Applicant: Consejo Superior de Investigaciones Cientificas (CSIC), 28006 Madrid (ES); Abiopep, S.L., 30100 Murcia (ES)
(72) Inventor: ARANDA REGULES, Miguel Ángel, 30100 Murcia (ES); NAVARRO SEMPERE, Raquel, 30100 Murcia (ES); GÓMEZ LÓPEZ, Pedro, 30100 Murcia (ES); AGÜERO GONZÁLEZ, Jesús, 30100 Murcia (ES); MÉNDEZ-COLMENERO, Antonio, 30100 Murcia (ES)
(74) Representative: Pons
(86) International application number: PCT/ES2016/070442
(87) International publication number: WO 2016/198722

(57) **Abstract**

The present invention relates to the use of two isolates of different strains of Pepino mosaic virus (PepMV) to protect a plant against infection by PepMV, wherein said isolates belong to different strains and interact antagonistically. Also described is a method for protecting a plant against infection by PepMV, as well as a kit that comprises the components necessary for implementing the invention.

## Description

The present invention relates to the use of two isolates of Pepino mosaic virus (PepMV) to protect a plant against infection by PepMV, wherein said isolates belong to different strains and interact antagonistically. Therefore, the present invention pertains to the field of agriculture, specifically the sector aimed at controlling pests and diseases that affect crops.

### STATE OF THE ART

Plant viruses are responsible for most health concerns in agriculture, not only because they lead to significant economic losses in the production sector, but also because of shortages and ineffective control measures that are available to fight these pathogens in agricultural crops. The methods for controlling virus-induced diseases include preventing the transmission thereof, applying hygiene measures during the propagation of plant material and during cultivation, using resistant varieties, and cross-protecting.

One virus that is causing most serious problems to tomato crops is Pepino mosaic virus (PepMV). PepMV belongs to genus *Potexvirus* and was first isolated in Peru from Solanum muricatum plants in 1974. PepMV was recognized as a tomato pathogen in the Netherlands in 1999 and since then, and during a period of four years, outbreaks of the disease caused by PepMV arose in the main tomato production areas of Europe and the Americas. PepMV may lead to a range of symptoms in tomatoes that comprise light green mosaics, bright yellow mosaics, leaf bubbling along with other distortions on the leaves, and green streaks on the stems. PepMV critically affects the yield and quality of fruits, so that it is currently considered a virus with significant agricultural relevance and is included on the European Plant Protection Organization (EPPO, 2011) alert list. In Europe, it is present in 19 countries and poses a serious threat to tomato production in at least the Netherlands, Belgium, Poland, France and Spain.

The molecular epidemiology of natural PepMV populations in Spain was studied by Gómez et al., 2009 (Journal of Virology, 83(23):12378-12387). These authors showed that populations of this virus in production areas of greenhouse tomatoes in the Murcia region were composed of isolates of two strains of the virus, the Chilean strain (CH2) and the European strain (EU) that cocirculated. They found that the CH2 isolate accumulated at a higher rate than the EU isolate in tomato plants infected with a representative isolate of the CH2 strain or with a representative isolate of the EU strain. However, when the two isolates coinfected the same plant, asymmetrical antagonism occurred among the isolates of both strains, such that the CH2 isolate then accumulated at a lower rate than the EU strain, while the accumulation levels of the latter remained unchanged. Likewise, they demonstrated that coinfection with both strains increased the range of PepMV hosts and that the coinfected plants remained symptomless for several weeks after inoculation. The authors noted that said lack of symptoms could represent a serious problem for farmers when preventing and managing the disease, since it is impossible to distinguish healthy plants from coinfected plants, these acting as reservoirs of the disease.

The methods for controlling PepMV-induced diseases essentially include destroying the infected plants and preventing the transmission of the virus by means of hygiene measures during the cultivation and propagation of plant material. It would be highly desirable to have commercial varieties of tomatoes that are resistant to PepMV; however, none of these varieties currently exist on the market and it is likely that they will not become available in the short term, since the few sources of resistance identified show specific partial resistance to the viral strain and inheritance as a polygenic trait.

Another alternative for controlling the disease may be cross protection. Viral cross protection in plants was described several decades ago (Hull, 2014. Plant Virology, Academic Press) and has been used in cases of phytosanitary emergencies such as, for example, that which was caused by the *Citrus tristeza virus* (CTV) in Brazil (Moreno et al., 2008. Molecular Plant Pathology, 9(2), 251-268). Cross protection (also known as pre-immunization) is based on infection with an attenuated isolate of the virus that only causes mild symptoms in the crop and prevents infection by a more virulent isolate of the same virus, which theoretically involves some phytopathological risks, but has been used successfully and should be considered for use when there are no effective means of control against serious problems (Hull, 2014, cited *ut supra).*

The possibility of using cross protection against infection by PepMV has been studied by some authors. Hanssen et al., 2010 (Plant Pathology, 59:13-21) found that protection against the CH2 strain was only effective by pre-inoculating the plants with an attenuated isolate of the same strain, since otherwise the symptoms of the disease increased. Likewise, they concluded that a strategy for controlling the disease based on cross protection can only be viable in areas where a strain of PepMV predominates, provided that the virus population is closely monitored and strict hygiene measures are implemented during cultivation and between the different cultivation cycles. Schenk et al., 2010 (Eur J Plant Pathol, 127:246-261) studied the potential for using attenuated isolates of PepMV for cross protection against infection with virulent isolates of European (EU) and Peruvian (PE) genotypes in tomato. These authors concluded that protection with attenuated isolates could only work under highly defined and controlled conditions. Furthermore, since the experiments were carried out under experimental greenhouse conditions, they noted that cross protection in the field cannot be effective due to the presence of numerous variants of the virus and that cross protection only worked well when there was high genetic proximity between the attenuated isolate and the aggressive isolate.

Therefore, the different approaches to cross protection against PepMV have not worked to date and cross protection methods must be developed against PepMV that allow for a broad spectrum of protection, prevent superinfection with more aggressive isolates of the virus, ensure protection under commercial cultivation conditions, and are reliable, safe and easy to apply.

### DESCRIPTION OF THE INVENTION

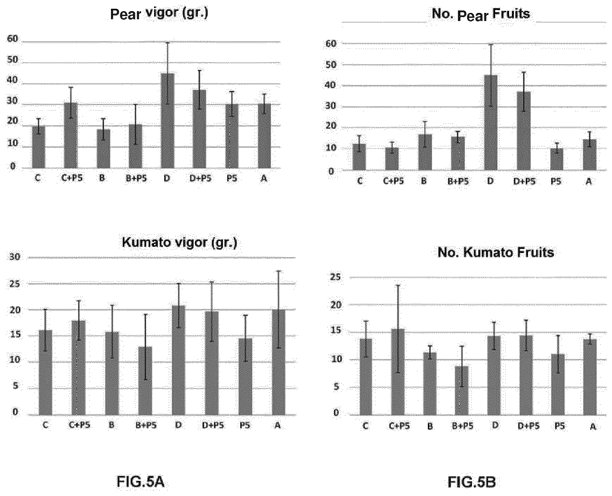

The inventors of the present invention have discovered that inoculating a tomato plant with two isolates of different strains of the Pepino mosaic virus (PepMV) that interact antagonistically prevent the plant from being infected by aggressive PepMV isolates, thus ensuring protection against PepMV populations in the field by providing a method of protection that is reliable, safe and easy to apply. As shown in Example 2, the inventors administered an inoculum to tomato plants that was obtained from the combination of isolates of two PepMV strains, the Chilean strain (CH2) and the European strain (EU), and they observed that the plants treated with this inoculum showed a greater vigor than that shown by the untreated plants, both plants subsequently infected with the aggressive isolate of PepMV (PepMV-P5) and those that were not infected (Figures 5A and 5B). Afterwards, these results were verified by assays in commercial cultivation fields of tomatoes (Examples 3 and 4).

In light of this fact, the inventors developed a series of inventive aspects that are described in detail below.

### Use of isolates of PepMV strains to provide protection against infection by PepMV

The present invention is based on the fact that infecting a plant with two isolates of different PepMV strains that interact antagonistically provides protection to said plant against infections by more aggressive isolates of PepMV. Therefore, the isolates of PepMV must have two characteristics: (1) belong to different strains, and (2) interact antagonistically. As known by a person skilled in the art, this way of providing protection is called "cross protection" and refers to inoculating a plant with a virus for the purpose of providing protection to said plant against a subsequent infection of the same virus, also known as superinfection exclusion. In this way, inoculating the plant with these two isolates prevents the infection of a virulent isolate of one strain or another that subsequently attacks said plant. In general, it is said that there is "cross protection" or "cross protecting" when more than 50% of the plants inoculated with the viral isolates are resistant to subsequent infection of the same virus (as described in Lecoq, H. (1998) "Control of plant virus diseases by cross protection" and in Hadidi A., Khetarpal R. K. and Koganezawa H. eds. Plant Virus Disease Control, APS Press, St. Paul, Minnesota, pp. 33-40).

Therefore, in one aspect, the invention relates to the use of an isolate of a first strain of the Pepino mosaic virus (PepMV) and an isolate of a second strain of PepMV to provide protection to a plant against infection by PepMV, in which said isolates:
(i) belong to different strains, and
(ii) interact antagonistically.

In the present invention, it is understood that a plant is protected against, or is resistant to, an infection by PepMV when the plant, after having been inoculated with isolates of PepMV, does not show symptoms or shows reduced or fewer common symptoms of an infection by PepMV, in other words, light green mosaic, bright yellow mosaic, leaf bubbling along with other distortions on the leaves, and green streaks on the stem, as well as an irregular distribution of pigments and other distortions on fruits that can make them lose their commercial value. Since inoculating a plant with both isolates provides protection against a subsequent infection of PepMV, said combination of isolates can also be called vaccine or pre-inoculum.

In the context of the present invention, it is understood that any two isolates belong to different strains of PepMV when the sequence identity between the genomes thereof is comprised between 80% and 92% (excluding 92%). On the other hand, it is understood that any two isolates belong to the same strain of PepMV when the sequence identity between the genomes thereof is comprised between 92% and 100% (including 92%).

In the present invention, "identity" or "sequence identity" is understood as the degree of similarity between two nucleotide sequences calculated by aligning the two sequences. A degree of identity expressed as a percentage will be calculated based on the number of common residues among the aligned sequences. The degree of identity between two amino acid sequences can be determined by conventional methods, for example, standard sequence alignment algorithms known in the state of the art, such as BLAST [Altschul S.F. et al. Basic local alignment search tool. J Mol Biol. 1990 Oct 5; 215(3):403-10]. The BLAST programs, for example, BLASTN, BLASTX, TBLASTX, BLASTP and TBLASTN are a part of the public domain on the National Center for Biotechnology Information (NCBI) website.

Moreover, another characteristic of the isolates used in the present invention is the required existence of antagonistic interaction between them. Interactions between plant viruses in mixed infections are generally classified as synergistic or antagonistic. A synergistic interaction has a facilitating effect on both viruses that is usually shown as an increase in viral replication in the host plant. Meanwhile, in an antagonistic interaction, one of the viruses usually benefits from (or is not affected by) the interaction and its presence reduces the replication rate and accumulation of the other virus (Syller, 2012. Molecular Plant Pathology, 13(2)204-216). Typically, the assay that is carried out to determine how two isolates interact consists of co-inoculating both isolates in plants, sampling these plants at different post-inoculation times, and determining the accumulation of the two isolates in these samples through quantitative assays, such as reverse transcription followed by real-time quantitative PCR.

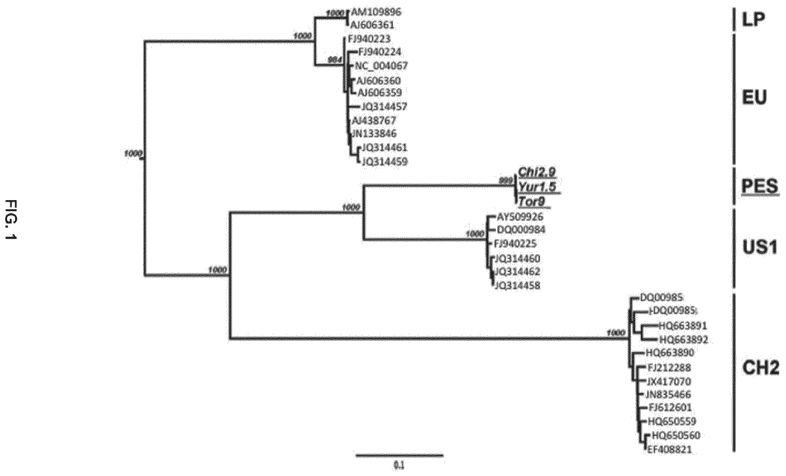

In the present invention, the strains belong to Pepino mosaic virus or PepMV. PepMV is a virus that belongs to the genus *Potexvirus* and its genome consists of a single positive-sense RNA chain with a size of 6.4kb. Using the aforementioned criteria, four strains of PepMV were described, which encompass the European (EU), original Peruvian (PE), Chilean (CH2), American (US) and South Peruvian (PES) genotypes (Moreno-Pérez et al., 2014, cited *ut supra)* (Figure 1). Each of these strains is formed by a group of individuals called isolates. The isolates of PepMV used in the present invention can belong to any of these strains. However, in a particular embodiment, the isolate of the first strain of PepMV belongs to the Chilean (CH2) genotype, and the isolate of the second strain of PepMV belongs to the European (EU), American (US), original Peruvian (PE) or South Peruvian (PES) genotype. In another more particular embodiment, the isolate of the first strain of PepMV belongs to the Chilean (CH2) genotype, and the isolate of the second strain of PepMV belongs to the European (EU) genotype.

Examples of isolates of the CH2 strain include, but are not limited to, PepMV-PS5, PepMV-KLP2 and PepMV-P5.

Examples of isolates of the EU strain include, but are not limited to, PepMV-Sp13, PepMV-LP2001 and PepMV-LI2000.

Examples of isolates of the US strain include, but are not limited to, PepMV-US1.

Examples of isolates of the PES strain include, but are not limited to, PepMV-Chi2.9, PepMV-Yur1.5 and PepMV-Tor9.

In a particular embodiment, the isolate of the first strain of PepMV of the Chilean genotype is the PepMV-PS5 isolate and/or the isolate of the second strain of PepMV of the European genotype is the PepMV-Sp13 isolate.

The PepMV-PS5 isolate was deposited in the German Collection of Microorganisms and Cell Cultures *(Leibniz Institute DSMZ-German Collection of Microorganisms and Cell Cultures*, Inhoffenstraβe 7B, 38124 Braunschweig, GERMANY) under the Budapest Treaty on June 4, 2015. The deposit number assigned was DSM32070.

The PepMV-Sp13 isolate was deposited in the German Collection of Microorganisms and Cell Cultures *(Leibniz Institute DSMZ-German Collection of Microorganisms and Cell Cultures*, Inhoffenstrape 7B, 38124 Braunschweig, GERMANY) under the Budapest Treaty on June 4, 2015. The deposit number assigned was DSM32069.

In accordance with the present invention, the genomes of these isolates share an identity less than 92% and they behave antagonistically, as demonstrated in Gómez et al., 2009. Journal of Virology, 83(23):12378- 12387.

As explained at the beginning of the present description, applying the isolates of the two different strains of PepMV to the plants, which interact antagonistically, provides protection against subsequent infections by PepMV. In the context of the present invention, the terms "application", "administration" and "inoculation" are equivalent and all imply that the isolates of the strains come in contact with the plants to be protected or vaccinated against infection by PepMV.

The inoculation of the isolates of the strains can be carried out by any method known in the state of the art. Examples of these methods of inoculation include, but are not limited to, spraying, soaking, prolonged immersion, etc.

The use of abrasives is a common practice in inoculation. The three common types of abrasives used are silicon carbide (carborundum), aluminum oxide (corundum) and diatomaceous earth (celite). The abrasive can be fine (sifted with a woven mesh of 300 to 600 threads per inch (118/cm)) and can be added to the inoculum or sprinkled over the leaves of the plant. The leaves should be rubbed lightly to avoid producing visible rubbing lesions on the leaves. The infection can sometimes be extended by rubbing the same surface three or four times instead of once.

One of the factors that predisposes plants to the viral infection is the state of the plant. In general, the best time of day to proceed with inoculation is the time that coincides with the highest turgidity of the cells, preferably shortly after irrigation.

As known by a person skilled in the art, when the number of plants to be inoculated is relatively low, the most common inoculation technique is to rub the juice of an infected plant onto the leaves of healthy plants. In a typical assay of mechanical transmission, the plants to be inoculated are sprinkled with an abrasive and the tissues of an infected plant are macerated with a mortar and pestle in a buffer solution, using 1 to 10 mL of the solution per gram of tissue. Next, the surface of the leaves of the plant to be inoculated is submerged in the inoculum and rubbed lightly with a gauze pad, cotton pad, brush, pestle, spatula, plant pot stick, etc. The leaves can be rinsed with water after inoculation, but this may increase, decrease or have no effect on the infection, depending on the virus-host and inoculum combination. After this, the plants are placed under suitable conditions (e.g. greenhouse) so that the inoculum colonizes the plant. This essential technique has many modification alternatives.

Alternatively, inoculation by high pressure spraying is the technique of choice when a large number of plants must be inoculated with a given virus. The spray gun is useful for inoculating individual plants; with this method, approximately 1% of fine carborundum (or another abrasive) is added to the inoculum and it is shaken to keep it in suspension during inoculation. Spraying is applied at a pressure between 60 and 80 Ibs/in² (psi) [413.6855 and 551.5807 KPa], and the nozzle is held 1-2 cm away from the surface of the leaf. Other types of sprayers have also been used successfully for mass inoculations in the field.

As understood by a person skilled in the art, a virus is an acellular microscopic infectious agent that can only multiply inside the cells of other organisms. Therefore, in the present invention, the isolates of the strains will be administered inside a cell called a "carrier" which, preferably, will be an extract of the plant material in which the virus has previously multiplied. The extract of plant material, carrier of the infectious inoculum of the isolates of each of the strains in question, can be used to inoculate the plants in different ratios to each other. Thus, the isolates of the strains can be used in a ratio of 1:1, 1:2, 1:3 or others, of one isolate to the other. However, in a particular embodiment, the plant is inoculated with the isolates of the strains in a ratio of 1:1 to each other; in other words, the same amount of infectious plant material, carrier of each isolate of each strain, is inoculated. However, the inventors have observed that when the isolates belong to the CH2 strain and the EU strain, respectively, the ratio of 1:2 provides greater protection against PepMV. Therefore, in a particular embodiment, the ratio of the isolate of the CH2 strain of PepMV (for example, PepMV-PS5) to the isolate of the EU strain of PepMV (for example, PepMV-Sp13) is 1:2.

The isolates can be administered to the plants simultaneously (at the same time) or sequentially (first one and then the other). Therefore, the isolates can be in the same composition or in separate compositions for the simultaneous or sequential administration thereof, respectively. Therefore, in a particular embodiment, the isolates are comprised in the same composition or in separate compositions.

As understood by a person skilled in the art, the concentration of the isolates in the composition can vary over a wide range, but always in a minimum concentration needed, or effective quantity, in order to produce infection. Said minimum concentration needed depends on the number of copies of the viral genome of each isolate. In the sense used in this description, the term "effective quantity" refers to the sufficient quantity to obtain beneficial results or the results desired. An effective quantity can be administered once or several times. In terms of treatment and protection, a "sufficient quantity" is the amount needed to alleviate, improve, stabilize, reverse, prevent, delay or slow down the infection of the plants by PepMV. It is a routine practice of a person skilled in the art to determine the minimum concentration needed based on the virus, the physiological state of the plant, etc.

Therefore, in a particular embodiment, the concentration of the isolates of each strain of PepMV in the composition is between 1x10⁵ and 5x10⁷ copies of the viral genome/µL. Any concentration of the isolates comprised within this interval is considered an effective quantity for producing the desired effect, in other words, to provide protection to a plant against infection by PepMV.

In another more particular embodiment, the concentration of the isolate of the strain of PepMV of the Chilean genotype in the composition is between 5x10⁵ and 5x10⁶ copies of the viral genome/µL, and/or the concentration of the isolate of the strain of PepMV of the European genotype in the composition is between 1x10⁶ and 1x10⁷ copies of the viral genome/µL. In another even more particular embodiment, the concentration of the isolate of the strain of PepMV of the Chilean genotype in the composition is 1x10⁶ copies of the viral genome/µL, and/or the concentration of the isolate of the strain of PepMV of the European genotype in the composition is 2x10⁶ copies of the viral genome/µL.

In addition to the viral isolates, the composition can comprise other components or constituents that facilitate the administration of the isolates to the plants, and/or that facilitate the infection, such as, but not limited to, the extract of infectious plant material, carrier of the isolates of the strains, an agriculturally acceptable vehicle, buffers, abrasives, solvents, growth regulators, etc., provided that they all allow or do not harm nor compromise the viability of the strains of PepMV.

The concentration of the extract of infectious plant material, carrier of the isolates of the strains, which ensures the minimum amount needed in the composition in order to provide protection to the plants is between 8 and 25 g/L, preferably a concentration of between 10 and 20 g/L, more preferably a concentration of 20 g/L.

In the sense used in this description, the term "agriculturally acceptable vehicle" is used to indicate that said vehicle comprises a substance or combination of substances that can be used in the agriculture sector, and includes any agriculturally acceptable liquid or solid material that can be added to and/or mixed with the isolates of the strains of PepMV (or with the extract of plant material, carrier of the isolates of the strains) in order to put them into a simpler or improved form of application, or with an applicable or desirable activation intensity. Due to the nature of the active ingredient of the composition (the isolates of PepMV), said agriculturally acceptable vehicle must allow or neither harm nor compromise the viability of said microorganism.

In a particular embodiment, the composition comprises an abrasive. The function of the abrasive and examples of abrasives have been explained in previous paragraphs.

The buffer class to be used in the inoculum (phosphate, borate, citrate, etc.) depends on the virus-host combination. The buffer solutions are usually used in concentrations of 0.01 M to 0.1 M and at pH 6 to 9. Some viruses require a reducing agent in the inoculum for good transmission. Sodium sulfite, sodium ascorbate and mercaptoethanol in concentrations of 0.1 to 1% can be added to the buffer solution to determine whether it improves transmission. Inhibiting oxidative enzymes such as sodium diethyldithiocarbamate in concentrations of 0.001 to 0.01 M have increased transmission in some cases. Some plants contain inhibitors of the infection, and transmission can be improved by diluting the inoculum. Transmitting a virus through plants with a high content of tannins is usually difficult, e.g. in the case of trees. Transmission can often be improved by grinding the infected tissue in 2% nicotine, 1% bentonite or the combination of both materials.

The composition of the invention can be used in solid or liquid form, for example, in the form of a wettable powder or an emulsifiable concentrate that incorporates the conventional diluents. Said compositions can be obtained in a traditional way, for example, by mixing the isolates of the strains of PepMV with a diluent and optionally with other formulation ingredients, such as those known by a person skilled in the art. The composition of the invention can be formulated in a way that is suitable for the administration thereof by spraying, sprinkling, or any other method of agricultural administration known in the state of the art.

Any plant can be object of the present invention, including monocotyledonous and dicotyledonous plants. In the present invention, the term "plant" includes complete plants, plant predecessors and progenies, and plant parts, including buds, stems, leaves, roots (including tubers), flowers, tissues and organs, which can be susceptible to viral infection.

The plants included in the present invention belong to the Viridiplantae superfamily. Examples of plants include, but are not limited to, *Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luff a acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsac[upsilon]m dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris* and *Ziziphus spp.,* among others.

Preferably, examples of plants object of the present invention include, but are not limited to, plants of agronomic interest, such as cereal plants (wheat, rye, barley, etc.), fruit trees, forage plants (for example, legumes, eggplants, peppers, melons, watermelons, cucumbers, zucchini, beans, peas, onions, Chinese cabbage, parsley, radishes, etc.), and ornamental plants.

In a particular embodiment, the plant of the present invention belongs to the *Solanum sp.* genus, in particular it is a tomato plant. The tomato plant of the present invention can be, for example, *Solanum lycopersicum, Solanum peruvianum, Solanum pimpinellifolium, Solanum lycopersicoides, Solanum sitiens, Solanum juglandifolium, Solanum ochranthum, Solanum cheesmaniae* or *Solanum galapagense.*

Preferably, the tomato plant is of the *Solanum lycopersicum* species, or synonyms thereof, *Lycopersicon esculentum, Lycopersicon esculentum* Mill., *Lycopersicon esculentum* var. *esculentum, Solanum esculentum, Solanum esculentum* Dunal. Tomato plant varieties include, but are not limited to, Anna Russian, Applause, Aussie, Baladre, Bella Rosa, Black Cherry, Black Russian, Blondkopfchen, Brandywine, Carbon, Ceylan, Cherokee Purple, Cherry, Cherry Redondo, Tomate Cherry Pear Amarillo, Comanche, Costoluto Genovese, Ditmarcher, Eros, Gallician, Glacier, Gartenperle, Green sausage, Grushovka, Harzfeuer, Hugh, Japanese Black, Jersey Devil, Kosovo, Krim Black, Kumato, Liguria, Limachino, Lime Green Salad, Manitoba, Marvel Stripe, Moneymaker, Muxamiel, Estrella, Opalka, RAF, Black Pear, Piña Hawaiana, Rio Grande, San Marzano, Siberian, Sprite, Sugary, Sun Sugar, Tigerella, White Queen, Raf Claudia, Roma, Valenciano, Pear de Girona, Montserrat, Corazón de Buey, Angela, Colgar en Rama, Ciruela Negro, Optima, Pata Negra, Copia, Velasco, Montenegro, Vertyco, Ventero, Ramyle, Pitenza, Paladium, Mayoral, Razymo, Motto, Caniles, Byelsa, Royalty, Trujillo, Delizia, Dumas Duratom, Larguero, Torry, Tovistar, Pintón, Grueso, Larga Vida, Marenza, Window Box Roma, Ninette, Retinto, Boludo, Anairis, Tobi Star, Myla, Guarapo, Atago, Jawara, Velasco, Manitu, Colbi, Duraton, Patriarca, Danubio, Intense, Pear Fitto, Vernal, Cecilio, Cherry Kumato, Cherry Amarillo, Cherry Redondo, Cherry Ministar, Cherry Guindos, Cherry Marinica and Cherry Angel.

In a particular embodiment, the plant is a tomato plant, preferably the tomato plant belongs to a tomato variety selected from the group consisting of the varieties of Caniles, Ventero, Intense, Pear Fitto, Ventero, Angele, Pitenza, Ninette, Retinto, Boludo, Anairis, Tobi Star, Myla, Guarapo, Atago, Velasco, Jawara, Muxamiel, Montenegro, Manitu, Colbi, Duraton, Patriarca, Danubio, Vernal, Cecilio, Kumato, Cherry Kumato, Cherry Amarillo, Cherry Redondo, Cherry Ministar, Cherry Guindos, Cherry Marinica and Cherry Angel.

### Method for providing protection against an infection by PepMV

As derived from the use described in the previous inventive aspect, the present invention also relates to a method for providing protection to a plant against infection by PepMV.

Therefore, in another aspect, the invention relates to a method for providing protection to a plant against infection by PepMV, hereinafter "method of the invention", which comprises inoculating said plant with an isolate of a first strain of PepMV and an isolate of a second strain of PepMV, in which said isolates:
(i) belong to different strains, and
(ii) interact antagonistically.

The terms and expressions used in the method of the invention have been described and explained in previous paragraphs and are applicable to the present inventive aspect.

In a particular embodiment, the isolate of the first strain of PepMV belongs to the Chilean (CH2) genotype, and the isolate of the second strain of PepMV belongs to the European (EU), American (US), original Peruvian (PE) or South Peruvian (PES) genotype. In another more particular embodiment, the isolate of the first strain of PepMV belongs to the Chilean (CH2) genotype, and the isolate of the second strain of PepMV belongs to the European (EU) genotype.

In another more particular embodiment, the isolate of the first strain of PepMV of the Chilean genotype is the PepMV-PS5 isolate and/or the isolate of the second strain of PepMV of the European genotype is the PepMV-Sp13 isolate.

In a first step, the method of the invention comprises inoculating a plant with an isolate of a first strain of PepMV and an isolate of a second strain of PepMV wherein said isolates (i) belong to different strains, and (ii) interact antagonistically. Techniques for inoculating the plants with the strains have been explained in the previous inventive aspect (use of the strains).

In a particular embodiment, the plant is inoculated with the isolates in a ratio of 1:1 of one isolate to the other, where, in a more particular embodiment, the plant is inoculated with an isolate of the CH2 genotype and an isolate of the EU genotype in a ratio of 1:2.

Moreover, inoculation can be carried out simultaneously or sequentially. In the present invention, simultaneous inoculation is understood as the inoculation of the two isolates at the same time; in other words, both isolates form part of the same composition that is going to be administered to the plant, or inoculation is carried out at the same time even if they form part of separate compositions. In the present invention, sequential inoculation is understood as the inoculation of plants with an isolate and then inoculation with the other isolate. As understood by a person skilled in the art, in this case both isolates will be formulated in separate compositions. In another particular embodiment, the isolates are comprised in the same composition or in separate compositions.

In a particular embodiment, the concentration of the isolates of each strain of PepMV in the composition is between 1x10⁵ and 5x10⁷ copies of the viral genome/µL. Any concentration of the isolates comprised within this interval is considered an effective quantity for producing the desired effect, in other words, to provide protection to a plant against infection by PepMV.

In another more particular embodiment, the concentration of the isolate of the strain of PepMV of the Chilean genotype in the composition is between 5x10⁵ and 5x10⁶ copies of the viral genome/µL, and/or the concentration of the isolate of the strain of PepMV of the European genotype in the composition is between 1x10⁶ and 1x10⁷ copies of the viral genome/µL. In another even more particular embodiment, the concentration of the isolate of the strain of PepMV of the Chilean genotype in the composition is 1x10⁶ copies of the viral genome/µL, and/or the concentration of the isolate of the strain of PepMV of the European genotype in the composition is 2x10⁶ copies of the viral genome/µL.

In another particular embodiment, the composition comprises an abrasive.

In a particular embodiment, the plant is a tomato plant, preferably the tomato plant belongs to a tomato variety selected from the group consisting of the varieties of Caniles, Ventero, Intense, Pear Fitto, Ventero, Angele, Pitenza, Ninette, Retinto, Boludo, Anairis, Tobi Star, Myla, Guarapo, Atago, Velasco, Jawara, Muxamiel, Montenegro, Manitu, Colbi, Duraton, Patriarca, Danubio, Vernal, Cecilio, Kumato, Cherry Kumato, Cherry Amarillo, Cherry Redondo, Cherry Ministar, Cherry Guindos, Cherry Marinica and Cherry Angel.

### Kit for providing protection against infections by PepMV and the use thereof

As understood by a person skilled in the art, the elements needed for implementing the invention are available to a person skilled in the art, for example, in the form of a kit.

Therefore, in another aspect, the invention relates to a kit, hereinafter "kit of the invention", which comprises:
- A plant extract that comprises a pure inoculum of an isolate of a first strain of PepMV, and
- A plant extract that comprises a pure inoculum of an isolate of a second strain of PepMV,
wherein both isolates (i) belong to different strains, and (ii) interact antagonistically.

From the kit of the invention, a person skilled in the art can prepare the inocula (or compositions) necessary for implementing the method of the invention and providing protection to plants against infection by PepMV. The preparation of said inocula or compositions for the administration thereof to the plants is a routine practice for a person skilled in the art. Moreover, the way of administering said inocula or compositions to the plants has been explained in the present description for other inventive aspects. The kit of the invention envisages all the particular embodiments described for the previous inventive aspects.

In a particular embodiment, the kit of the invention further comprises an abrasive. Examples of abrasives have been previously described in the present description.

In a particular embodiment, the isolate of the first strain of PepMV belongs to the Chilean (CH2) genotype, and the isolate of the second strain of PepMV belongs to the European (EU), American (US), original Peruvian (PE) or South Peruvian (PES) genotype. In another more particular embodiment, the isolate of the first strain of PepMV belongs to the Chilean (CH2) genotype, and the isolate of the second strain of PepMV belongs to the European (EU) genotype.

In another more particular embodiment, the isolate of the first strain of PepMV of the Chilean genotype is the PepMV-PS5 isolate and/or the isolate of the second strain of PepMV of the European genotype is the PepMV-Sp13 isolate.

In another aspect, the invention relates to the use of a kit for providing protection to a plant against infection by PepMV.

In a particular embodiment, the plant is a tomato plant, preferably the tomato plant belongs to a tomato variety selected from the group consisting of the varieties of Caniles, Ventero, Intense, Pear Fitto, Ventero, Angele, Pitenza, Ninette, Retinto, Boludo, Anairis, Tobi Star, Myla, Guarapo, Atago, Velasco, Jawara, Muxamiel, Montenegro, Manitu, Colbi, Duraton, Patriarca, Danubio, Vernal, Cecilio, Kumato, Cherry Kumato, Cherry Amarillo, Cherry Redondo, Cherry Ministar, Cherry Guindos, Cherry Marinica and Cherry Angel, or any other commercial tomato variety.

Throughout the description and in the claims, the word "comprises" and its variants are not intended to exclude other technical characteristics, additives, components or steps. For those skilled in the art, other objects, advantages and characteristics of the invention may be deduced from both the description and the practical use of the invention. The following examples and drawings are provided by way of illustration, and are not meant to limit the present invention.

### BRIEF DESCRIPTION OF THE FIGURES

**Figure 1** shows a reconstruction of the phylogenetic relationship among isolates of PepMV. Phylogenetic tree of maximum likelihood based on the nucleotide sequence of the complete genome of 30 isolates of the strains of PepMV of the 4 genotypes described (CH2, US1, EU, LP) and three isolates (Yur1.5, Tor9 and Ch2.9) of the strain of the PES genotype. The latter are underlined in the tree. The statistical support of each node was determined by a bootstrap analysis with 1,000 repetitions. Taken from Moreno-Pérez et al. 2014, Journal of Virology, 88(6):3359-3368).

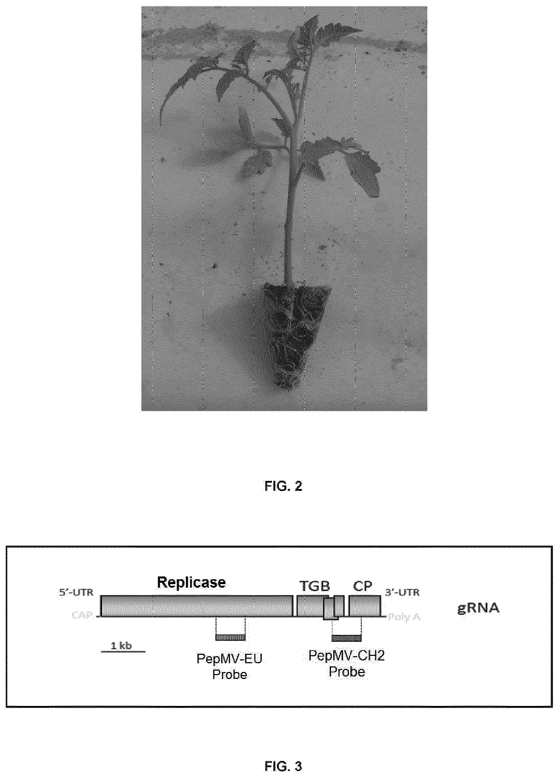

**Figure 2** shows the phenological state of a tomato plant at the time of transplant thereof from the seedbed to the commercial greenhouse.
**Figure 3** shows the genome of PepMV. The fragments of the genome used as probes to distinguish isolates of the EU strain from the CH2 strain are indicated.

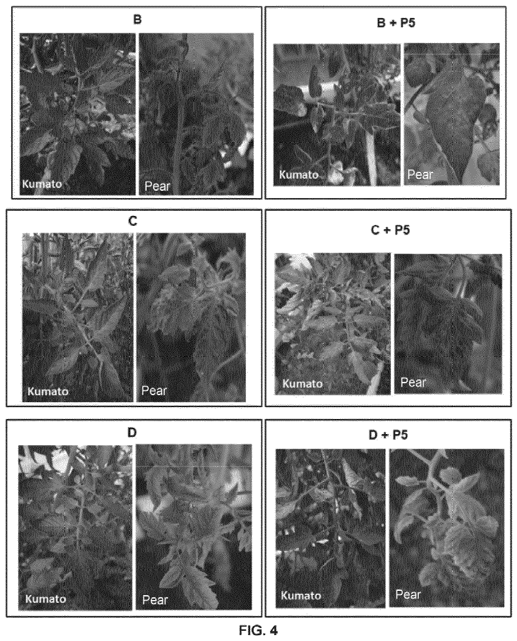

**Figure 4** shows the effect of pre-immunizations on tomatoes with inocula of the EU strain (PepMV-Sp13), the CH2 strain (PepMV-PS5) and the product object of the present invention in superinfection with the PepMV-PS5 virulent isolate. Left panel: A: Healthy control; B: inoculated with the isolate of the European (EU) strain (PepMV-Sp13); C: inoculated with the isolate of the Chilean (CH2) strain (PepMV-PS5); and D: inoculated with the product object of the present invention mixture of isolates of the European (EU) strain (PepMV-Sp13) and the Chilean (CH2) strain (PepMV-PS5). Right panel: The same treatments superinfected with aggressive isolate of the CH2 strain, PepMV-P5.
**Figures 5A and 5B** show the quantification of the effect of the pre-immunization treatments with isolates of the EU strain (PepMV-Sp13) and CH2 strain (PepMV-PS5) and the product of the invention in superinfection with the PepMV-P5 virulent isolate. Figure 5A shows the effect in vigor and the production of tomatoes of the Pear variety, and 5B shows the effect in vigor and the production of tomatoes of the Kumato variety. A: Healthy control; B: inoculated with the isolate of the European (EU) strain (PepMV-Sp13); C: inoculated with the isolate of the Chilean (CH2) strain (PepMV-PS5); D: inoculated with the product object of the present invention mixture of isolates of the European (EU) strain (PepMV-Sp13) and the Chilean (CH2) strain (PepMV-PS5). P5: Healthy plant superinfected with the aggressive isolate of the CH2 strain, PepMV-P5, B+P5, C+P5 and D+P5 are the treatments A, B, C and D superinfected with the aggressive isolate of the CH2 strain PepMV-P5.

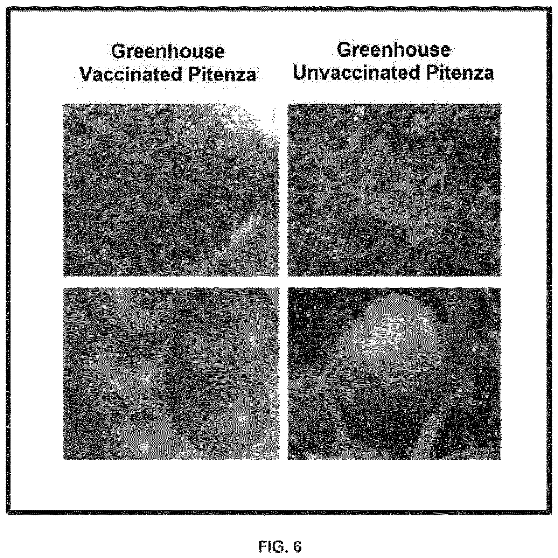

**Figure 6** shows the result of pre-immunization with the product of the invention under production conditions in a commercial greenhouse.

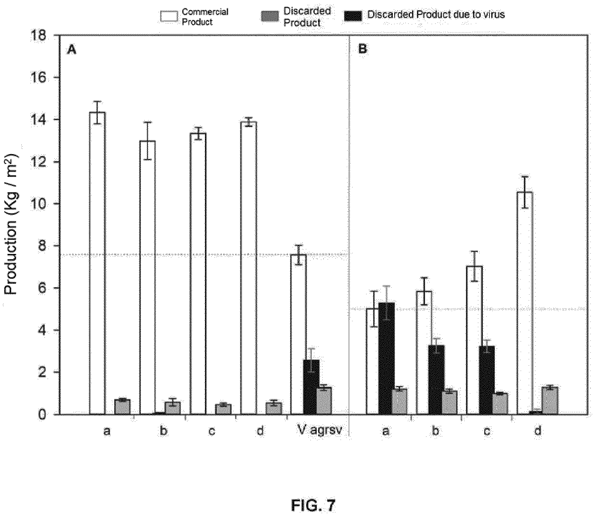

**Figure 7** shows a bar graph that shows the effectiveness of the inoculation with the isolates of the EU strain (PepMV-Sp13) and CH2 strain (PepMV-PS5) when providing protection to tomato plants against a more aggressive infection by PepMV (PepMV-KLP2). Key: (a) healthy plant control; (b) isolate of the EU strain (PepMV-Sp13); (c) isolate of the CH2 strain (PepMV-PS5); (d) inoculation with the product object of the present invention; V agresv in the left panel means the plants have been pre-inoculated with an aggressive virus (PepMV-KLP2) as a control at the same time that they were pre-inoculated with other strains of the assay; the right panel shows inoculation of the plants with the PepMV-KLP2 aggressive virus as a challenge to the functioning of the different treatments two weeks after the prior pre-inoculation. Commercial product: product (tomato) of commercial quality; Discarded product: nonspecific discarded product (tomato) and Discarded product due to virus: discarded product due to virus symptoms.

### EXAMPLES

The invention is illustrated below by means of assays carried out by the inventors which reveals the effectiveness of the invention claimed herein.

### MATERIALS AND METHODS.

### 1. Strains, viral isolates and plant material

The isolates of the viral strains used in the present infection, PepMV-Sp13 isolate of the European (EU) strain and PepMV-PS5 isolate of the Chilean (CH2) strain, and PepMV-P5, PepMV-CS10 and PepMV-KLP2 viral isolates of the CH2 strain are maintained on the infected and freeze-dried *Nicotiana benthamiana* material (leaves with symptoms) that are conserved at 4°C (Aguilar et al., 2002. Archives of Virology, 147(10), 2009-2015).

The *N. benthamiana* seeds are treated with 10 mg/mL of gibberellin in the night darkness and at room temperature; next, they are planted in seedbed trays with 294 cells and are maintained in a greenhouse under day and night conditions for 16 hours at 25-26°C and 8 hours at 19°C, respectively; after 10 days, they are transplanted to trays with 24 pots and are maintained in a greenhouse; 25-26 days after planting (4-5 true leaves), they are inoculated with the isolate of the corresponding strain of the virus and are maintained in the greenhouse until the leaves that show symptoms are collected. The leaves that show symptoms are collected, cut into strips, introduced into duly labelled paper envelopes, and freeze-dried.

The tomato plants used in the present invention come from commercial seedbeds and have 5-6 true leaves (Figure 2), following common sector procedures.

### 2. Preparation of probes and tissue print hybridization

The probes used to check the purity of the inocula of each isolate of the strains of PepMV of the present invention (PepMV-Sp13 of the European strain and PepMV-PS5 of the Chilean strain) were obtained by *in vitro* transcription of the DNA inserted into plasmids described in the state of the art (Sempere et al., 2011, Plant Methods 2011,7:6 and Spanish patent ES2351917B1).

The probes were prepared by techniques known in the state of the art that use DIG-11-UTP to mark the isolated sequences as described in (Sempere et al., 2011, Plant Methods 2011,7:6 and Spanish patent ES2351917B1) and common molecular biology procedures.

The tissue-print analysis of the samples was performed on leaf petiole imprints on positively loaded nylon membranes (Roche). The process of hybridization and detection by chemiluminescence was carried out by essentially following the protocol proposed by Roche (RNA labeling and detection kit) as described in the Spanish patent ES2351917B1.

### Example 1. Preparation of inocula

The isolates of the EU strain (PepMV-Sp13) and CH2 strain (PepMV-PS5) were initially obtained by the inventors in samples taken in tomato greenhouses of commercial cultivation in two regions of the province of Murcia (Mazarrón and Águilas) from year 2000 to year 2008 (Aguilar et al., 2002, Archives in Virology 147: 2009-2015; Gómez et al., 2009. Journal of Virology 83(23) 12378-12387). As of this time, pure cultures of the isolates of each of the two strains in freeze-dried plant material are maintained.
To prepare the pure inocula, the following steps were followed:
1. Generate the pure inocula of the isolates of each strain of the virus
2. Multiply the pure inoculum of the isolates of each strain of the virus
   1. Generate the pure inocula of the isolates of each strain of the virus using the pure cultures. The entire generation process of the pure inocula was carried out separately for each of the two strains. The pure culture of the isolates of each of the two strains was homogenized in the presence of a buffer that stabilizes the viral particles, such as a 30 mM phosphate buffer at pH=8 in a porcelain mortar with a ratio of 50 mg of plant material to 2 mL of buffer. This homogenized result was used to inoculate 25-26-day old *N. benthamiana* leaves (4-5 true leaves). To do so, the leaves to be inoculated were first sprinkled with an abrasive material, such as carborundum, and then they were rubbed with the homogenized result by hand. The plants thus inoculated with the isolate of each of the viral strains were maintained separately in a greenhouse kept under controlled conditions. After 10 and 15 days, the leaves that showed obvious symptoms of infection by PepMV were collected and the pure inoculum of the isolates of each of the EU and CH2 strains was obtained. These pure inocula consisting of infected *N*. *benthamiana* leaves and showing symptoms were kept separately at 4°C for up to 4 days for the processing thereof.
   2. Multiplication. The recently prepared pure inocula of the isolates of each viral strain were used to produce a greater quantity of inocula and generate stocks and the isolate of each strain was handled separately at all times. To do so, they were ground in the presence of a buffer for the purpose of stabilizing the viral particles, such as a 30 mM phosphate buffer at pH=8 at a concentration of 10 g/L. Next, a number of *N. benthamiana* plants (25-26 days old) were inoculated according to the quantity of inocula needed. Mass inoculation was carried out by applying pressure, such as by means of an airbrush. The airbrush consists of pressure spraying the inoculum on the plants at an adequate and sufficient distance, such as 20-30 centimeters, by using a source that generates pressure, such as a compressor, and an application system, such as a gravity feed airbrush gun. The output pressure of the gun is regulated to an adequate and sufficient pressure, such as 60-80 psi [413.6855 and 551.5807 KPa], in particular, 75 psi [517.1069 KPa]. To ensure greater effectiveness of the inoculation, an abrasive agent was added, such as carborundum (silicon carbide), into the gun chamber and was homogenized with the inoculum.

The plants inoculated with the isolates of each of the viral strains were maintained separately in a greenhouse. 10 to 14 days after inoculation, the infected leaves were collected by cutting above the inoculated leaves. The amplified pure inoculum was obtained by separately grinding the leaves infected with each viral strain in a buffer that stabilizes the viral particles, such as a 30 mM phosphate buffer at pH=8 at a concentration of 160 g/L, and preserved frozen at -80°C.

Based on the quantities of product needed, it is possible to carry out a second multiplication step of the two viral strains in *N. benthamiana* after initially multiplying the pure inoculum frozen at -80°C. This second multiplication step is carried out separately for each of the two strains and uses the inoculum obtained from the first multiplication step by defrosting at 4°C and proceeding as described in section 2 on Multiplication.

The purity of the inocula was checked by means of tissue-print hybridization, using specific probes for the EU and CH2 strains (Figure 3).

The product of cross protection object of the present invention was prepared by mixing equivalent quantities of inocula of each of the isolates of the EU and CH2 strains, obtained from a second multiplication step at a concentration of 20 g/L in buffer that stabilizes the viral particles, such as a 30 mM phosphate buffer at pH 8.

### Example 2. Checking the effectiveness of the product object of the present invention

The effectiveness of the product object of the present invention in cross protection against aggressive isolates of PepMV was checked in a greenhouse under controlled conditions. To do so, a PepMV-P5 aggressive isolate that leads to bright yellow spots on leaves and marbling on fruits was used. Three different pre-immunization treatments were applied:
(1) inoculum prepared from the isolate of the EU strain (PepMV-Sp13)
(2) inoculum prepared from the isolate of the CH2 strain (PepMV-PS5)
(3) inoculum prepared from the isolate of the CH2 strain (PepMV-PS5) and the EU strain (PepMV-Sp13) (hereinafter, "product of the invention").

Two commercial varieties of tomato were used (Pear and Kumato). To do so, immediately after the transplant from the seedbed (5-6 true leaves, Figure 2), seedlings of each of these two varieties were manually inoculated with each of the pre-immunization treatments indicated at a concentration of 20 g/L, with the help of an abrasive agent such as carborundum (silicon carbide). Each treatment was applied to two batches of 6 plants of each variety and two batches of another 6 plants were left untreated as the control of each variety.

Five weeks after applying the treatments, mechanical inoculation with the PepMV-P5 isolate, prepared as indicated in section 1 of Example 1, was carried out on one of the plant batches of each of the (pre-immunized) treatments. As a positive control, healthy plants with the same phenological state as the previously treated plants of one of the two batches per variety that were not treated, were inoculated with this isolate, leaving the second batch as a control. All plants were maintained in the greenhouse for a period of three months and symptoms were recorded every 7 days.

One month after inoculation with PepMV-P5, when the plants had already produced 4 clusters, the apical part immediately above the fourth tomato cluster was removed, and the fresh weight of the leaf immediately above said cluster was measured. The fresh weight of said leaf was used as an indicator of the vegetative vigor of the plant. Once the plants entered into production, two months after inoculation with PepMV-P5, the collection and characterization of the fruits began. This collection lasted for one month after entry into production.

No plant treated with the inoculum obtained by mixing the inocula of each of the two strains showed symptoms of bright yellow spots on the leaves thereof (Figure 4), regardless of the tomato variety. The plants that were not pre-immunized and the plants only treated with the isolate of the EU strain or the CH2 strain showed obvious symptoms of infection by PepMV-P5 (Figure 4, right panel). It is worth noting that although the varieties studied (Pear and Kumato) responded to the treatments carried out in different ways (Figure 5A and Figure 5B), in both cases treatment with the product of the invention did not have any negative effect in terms of vigor or production.

The Pear variety was shown to be more susceptible to infection by PepMV, developing more intense symptoms of PepMV on both the leaf and the fruit, depending on the treatment with the isolate of the EU strain or CH2 strain or with the most virulent PepMV-P5 isolate. However, the plants that were pre-immunized with the treatment object of the present invention did not show symptoms on leaves or on fruits. Furthermore, it is worth noting that the plants that were pre-immunized with the treatment object of the present invention showed a vigor greater than that shown in the control plants, the plants superinfected with the most virulent PepMV-P5 isolate and the plants that were not superinfected (Figures 5A and 5B).

On the other hand, the Kumato variety was more tolerant to the treatments carried out, showed less symptoms than the Pear variety on leaves and on fruits, and there were no significant differences compared to the plants that were not pre-immunized. However, in this variety, the plants that were pre-immunized with the treatment object of the present invention, both the batch superinfected with the most virulent isolate and the batch that was not superinfected, showed a vigor similar to the batch of control plants, which was greater than any other treatment, and a production level in the number of fruits equivalent to the control plants (Figures 5A and 5B).

### Example 3. Evaluation of the effectiveness of the product of the invention in commercial cultivation

The capacity of the product object of the present invention to protect against strains of PepMV present in tomato cultivation areas was evaluated in a 2 Ha commercial greenhouse located in Mazarrón, Murcia, an area especially affected by infection by PepMV.

The tomato variety used was Pitenza, a variety described as very susceptible to PepMV. The product of the present invention was applied to the tomato seedlings recently taken from the seedbeds (5-6 true leaves, Figure 2) at the time of final planting in the greenhouse of the crop.

The product was applied to the tomato seedlings at a concentration of 20 g/L by airbrush by using a compressor and gravity feed airbrush gun; the output pressure of the gun was regulated at 75 psi, in a quantity of approximately 5-7 liters per 10,000 of treated tomato plants; 12,000 plants/hour were inoculated by these means. A greenhouse of untreated Pitenza tomatoes located in front of the treated greenhouse was used as a control.

One month after applying the product of the present invention, samples were taken for the purpose of determining the percentage of pre-immunized plants, checking that the method of application is effective by achieving 100% pre-immunized plants. Likewise, the symptoms of PepMV in fruits in both greenhouses were evaluated. The evaluation made it possible to verify that 80-90% of the tomato fruits in the untreated greenhouse showed symptoms, while only 20-30% of those located in the treated greenhouse showed symptoms, which implies a reduction in the percentage of affected fruit by 60% (Figure 6). Last but not least, it was verified that treatment with the product of the invention did not have a negative effect in terms of vigor of the plants and production of the tomato fruits (Figure 6).

Experiments similar to those described for the Pitenza variety were conducted with a very significant number of commercial varieties of tomatoes with results equal to those obtained with said variety. Table 1 indicates the varieties of tomatoes assayed.

**Table 1. Tomato varieties assayed.**

| **Tomato varieties** | | | | |
|---|---|---|---|---|
| **Canary Type** | **Salad** | **Pear** | **Others** | **Cherry** |
| Ninette | Guarapo | Caniles | Vernal | Cherry Kumato |
| Retinto | Atago | Intense | Cecilio | Cherry Amarillo |
| Boludo | Velasco | Pear Fitto | Kumato | Cherry Redondo |
| Anairis | Jawara | | | Cherry Ministar |
| Tobi Star | Muxamiel | | | Cherry Guindos |
| Myla | Montenegro | | | Cherry Marinica |
| Pitenza | Manitu | | | Cherry Angel |
| | Colbi | | | |
| | Duraton | | | |
| | Partiarca | | | |
| | Ventero | | | |
| | Danubio | | | |

The experiments of the present example make it possible to conclude that under conditions of the commercial cultivation fields of tomatoes (i) the developed method of inoculation is very effective (12,000 plants/hour) and (ii) protection against superinfection of PepMV of the product object of the invention is also very effective (100% in the cases analyzed quantitatively).

### Example 4. Large-scale effectiveness assay of the product object of the present invention

Once the effectiveness of the product in a research greenhouse under controlled conditions is verified and in order to complete the information obtained in the evaluation assays on commercial cultivation, an assay was proposed to evaluate the effectiveness of the product object of the invention under large-scale production conditions against superinfection with aggressive PepMV isolates. The effectiveness of the product was assayed on tomato crops of 3 commercial varieties; PepMV-CS10 and PepMV-KLP2 isolates, respectively, which were isolated in 2014 by the inventors, were used as aggressive isolates. The commercial tomato varieties used were Angelle (Syngenta), Ventero (Monsanto) and Caniles (Zeraim Ibérica), and a total of 1,848 plants were used in a 2,400m² multi-tunnel greenhouse.

Three groups were established with the following treatments applied at the time of transplant: (a) control without pre-inoculation; (b) pre-inoculation with the PepMV-EU isolate of the European strain; (c) pre-inoculation with the PepMV-CH2 isolate of the Chilean strain; and (d) pre-inoculation with the product object of the invention. Two weeks after pre-inoculating them, two of the groups (2 and 3) were inoculated with the aggressive isolates of the CH2 strain, PepMV-CS10, which leads to yellow spots and lumps on the fruits of the infected plants, and PepMV-KLP2, which leads to yellow spots, very noticeable veins and necrosis in the fruits, respectively, and group 1 remained as the control with the four pre-inoculation treatments. Furthermore, pre-inoculation was carried out with the PepMV-KLP2 aggressive isolate at the time of transplant. Four repetitions per treatment and eight plants per repetition were carried out.

The symptoms on the plants were evaluated 16, 35, 68 and 103 days after inoculation with the aggressive isolates indicated, observing bright yellow mottling on all the plants of the treatments (a) (control without inoculation) and (b) (inoculated with the PepMV-EU strain), while symptoms were not observed with the treatment (c) (PepMV-CH2 strain) and the plants treated with the product object of the invention (d).

Tomato production was evaluated by gathering data from the different collections; the first collection took place 3 months after the start of the assay, and successive collections took place every 2-3 weeks at the beginning and every week in the final phase of the assay. Data on fruit production, size, number and characteristics was recorded for the commercial product (tomato suitable for the market), discarded product due to PepMV symptoms on the fruit (irregular maturation, necrosis, spots), and the discarded product (discarded due to unspecific low quality that is not clearly attributable to virus symptoms). The results showed that the plants of group 1, treatments (a), (b), (c) and (d) did not show significant differences in commercial tomato production (Figure 7, left panel), whereas the plants of groups 2 and 3, treatments (a), (b) and (c) not only showed symptoms, but also had a quantity of commercial product much lower than that of group 1, an important part of the product showed symptoms of PepMV and there was an increase in discarded products (Figure 7, right panel).

Furthermore, the treatment with the product object of the invention (d) does not lead to a decrease in the plant productivity, since production with the 3 treatments (b), (c) and (d) is similar to that of the control plant. However, the aggressive virus does lead to losses of approximately 50% in the planting, such that the crop loses its profitability (Figure 7, left panel). However, when the plants are artificially infected with the aggressive virus under controlled conditions, only the plants treated with the product object of the invention (d) maintain a level of production within the profit margins of the crop, since the production is around 75% of healthy plants and without any treatment (Figure 7, right panel). In the statistical study, the differences are significant with p<0.001. The data for each treatment was converted into log₁₀ to obtain normal distribution. The averages of each treatment were compared in pairs by means of the Student's t-test with a significance level of 5%.

The results of the present assay make it possible to conclude that under conditions of commercial cultivation fields of tomatoes, the protection of the product object of the present invention against superinfections with aggressive isolates of PepMV is very effective and makes it possible to restore production to levels close to the healthy control.

The previous examples demonstrate that the product object of the present invention ensures a broad spectrum of protection, prevents superinfection with more aggressive isolates of the virus, ensures protection under commercial cultivation conditions, and is reliable, safe and easy to apply.

## Claims

1. A use of an isolate of a first strain of Pepino mosaic virus (PepMV) and an isolate of a second strain of PepMV to provide protection to a plant against infection by PepMV, wherein said isolates:
(i) belong to different strains, and
(ii) interact antagonistically.

2. The use according to claim 1, wherein the isolate of the first strain of PepMV belongs to the Chilean (CH2) genotype, and the isolate of the second strain of PepMV belongs to the European (EU), American (US), original Peruvian (PE) or South Peruvian (PES) genotype.

3. The use according to claim 2, wherein the isolate of the first strain of PepMV of the Chilean (CH2) genotype is the PepMV-PS5 isolate with deposit number DSM32070.

4. The use according to claims 2 or 3, wherein the isolate of the second strain of PepMV of the European (EU) genotype is the PepMV-Sp13 isolate with deposit number DSM32069.

5. The use according to any of the claims 1 to 4, which comprises the inoculation of the plant with the isolates of the strains in a ratio of 1:1 to each other.

6. The use according to claims 2 to 4, which comprises the inoculation of the plant with an isolate of the Chilean (CH2) genotype and an isolate of the European (EU) genotype in a ratio of 1:2.

7. The use according to any of the claims 1 to 6, wherein the isolates are comprised in the same composition or in separate compositions.

8. The use according to claim 7, wherein the concentration of the isolates of each strain of PepMV in the composition is between 1x10⁵ and 5x10⁷ copies of the viral genome/µL.

9. The use according to claim 8, wherein the concentration of the isolate of PepMV of the Chilean (CH2) genotype in the composition is between 5x10⁵ and 5x10⁶ copies of the viral genome/µL, and/or the concentration of the isolate of PepMV of the European (EU) genotype in the composition is between 1x10⁶ and 1x10⁷ copies of the viral genome/µL.

10. The use according to claim 9, wherein the concentration of the isolate of PepMV of the Chilean (CH2) genotype in the composition is 1x10⁶ copies of the viral genome/µL, and/or the concentration of the isolate of PepMV of the European (EU) genotype in the composition is 2x10⁶ copies of the viral genome/µL.

11. The use according to any of the claims 7 to 10, wherein the composition comprises an abrasive.

12. The use according to any of the claims 1 to 11, wherein the plant is a tomato plant.

13. The use according to claim 12, wherein the tomato plant belongs to a tomato variety selected from the group consisting of the varieties of Caniles, Intense, Pear Fitto, Ventero, Angele, Pitenza, Ninette, Retinto, Boludo, Anairis, Tobi Star, Myla, Guarapo, Atago, Velasco, Jawara, Muxamiel, Montenegro, Manitu, Colbi, Duraton, Patriarca, Danubio, Vernal, Cecilio, Kumato, Cherry Kumato, Cherry Amarillo, Cherry Redondo, Cherry Ministar, Cherry Guindos, Cherry Marinica and Cherry Angel.

14. A method for providing protection to a plant against infection by PepMV, which comprises inoculating said plant with an isolate of a first strain of PepMV and an isolate of a second strain of PepMV, wherein said isolates:
(i) belong to different strains, and
(ii) interact antagonistically.

15. The method according to claim 14, wherein the isolate of the first strain of PepMV belongs to the Chilean (CH2) genotype, and the isolate of the second strain of PepMV belongs to the European (EU), American (US), original Peruvian (PE) or South Peruvian (PES) genotype.

16. The method according to claim 15, wherein the isolate of the first strain of PepMV of the Chilean (CH2) genotype is the PepMV-PS5 isolate with deposit number DSM32070.

17. The method according to claims 15 or 16, wherein the isolate of the second strain of PepMV of the European (EU) genotype is the PepMV-Sp13 isolate with deposit number DSM32069.

18. The method according to any of the claims 14 to 17, in which the inoculation of the plant with the isolates of the strains is carried out in a ratio of 1:1 to each other.

19. The method according to claims 15 to 18, wherein the inoculation of the plant with an isolate of the Chilean (CH2) genotype and an isolate of the European (EU) genotype is carried out in a ratio of 1:2.

20. The method according to any of the claims 14 to 19, wherein the inoculation of the plant with the isolates is carried out simultaneously or sequentially.

21. The method according to any of the claims 14 to 20, wherein the isolates are comprised in the same composition or in separate compositions.

22. The method according to claim 21, wherein the concentration of the isolates of each strain of PepMV in the composition is between 1x10⁵ and 5x10⁷ copies of the viral genome/µL.

23. The method according to claim 22, wherein the concentration of the isolate of PepMV of the Chilean (CH2) genotype in the composition is between 5x10⁵ and 5x10⁶ copies of the viral genome/µL, and/or the concentration of the isolate of PepMV of the European (EU) genotype in the composition is between 1x10⁶ and 1x10⁷ copies of the viral genome/µL.

24. The method according to claim 23, wherein the concentration of the isolate of PepMV of the Chilean (CH2) genotype in the composition is 1x10⁶ copies of the viral genome/µL, and/or the concentration of the isolate of PepMV of the European (EU) genotype in the composition is 2x10⁶ copies of the viral genome/µL.

25. The method according to any of the claims 21 to 24, wherein the composition comprises an abrasive.

26. The method according to any of the claims 14 to 25, wherein the plant is a tomato plant.

27. The method according to claim 26, wherein the tomato plant belongs to a tomato variety selected from the group consisting of the varieties of Caniles, Intense, Pear Fitto, Ventero, Angele, Pitenza, Ninette, Retinto, Boludo, Anairis, Tobi Star, Myla, Guarapo, Atago, Velasco, Jawara, Muxamiel, Montenegro, Manitu, Colbi, Duraton, Patriarca, Danubio, Vernal, Cecilio, Kumato, Cherry Kumato, Cherry Amarillo, Cherry Redondo, Cherry Ministar, Cherry Guindos, Cherry Marinica and Cherry Angel.

28. A kit that comprises:
- A plant extract that comprises a pure inoculum of an isolate of a first strain of PepMV, and
- A plant extract that comprises a pure inoculum of an isolate of a second strain of PepMV,
wherein both isolates (i) belong to different strains, and (ii) interact antagonistically.

29. The kit according to claim 28, which further comprises an abrasive.

30. The kit according to claims 28 or 29, wherein the isolate of the first strain of PepMV belongs to the Chilean (CH2) genotype, and the isolate of the second strain of PepMV belongs to the European (EU), American (US), original Peruvian (PE) or South Peruvian (PES) genotype.

31. The kit according to claim 30, wherein the isolate of the first strain of PepMV of the Chilean (CH2) genotype is the PepMV-PS5 isolate with deposit number DSM32070.

32. The kit according to claims 30 or 31, in which the isolate of the second strain of PepMV of the European (EU) genotype is the PepMV-Sp13 isolate with deposit number DSM32069.

33. A use of a kit according to any of the claims 28 to 32 to provide protection to a plant against infection by PepMV.

34. The use according to claim 33, wherein the plant is a tomato plant.

35. The use according to claim 34, wherein the tomato plant belongs to a tomato variety selected from the group consisting of the varieties of Caniles, Intense, Pear Fitto, Ventero, Angele, Pitenza, Ninette, Retinto, Boludo, Anairis, Tobi Star, Myla, Guarapo, Atago, Velasco, Jawara, Muxamiel, Montenegro, Manitu, Colbi, Duraton, Patriarca, Danubio, Vernal, Cecilio, Kumato, Cherry Kumato, Cherry Amarillo, Cherry Redondo, Cherry Ministar, Cherry Guindos, Cherry Marinica and Cherry Angel.
